(19) Europäisches Patentamt
European Patent Office
Office européen des brevets

(11) Publication number: **0 205 067**
**A2**

(12) # EUROPEAN PATENT APPLICATION

(21) Application number: 86107479.7

(22) Date of filing: 02.06.86

(51) Int. Cl.[4]: C 07 D 417/04
C 07 D 413/04, A 01 N 43/78
A 01 N 43/76

(30) Priority: 14.06.85 US 744728
18.02.86 US 830431

(43) Date of publication of application:
17.12.86 Bulletin 86/51

(84) Designated Contracting States:
DE FR GB IT

(71) Applicant: PPG INDUSTRIES, INC.
One PPG Place
Pittsburgh Pennsylvania 15272(US)

(72) Inventor: Schwindeman, James Anthony
295 Kenridge Road
Fairlawn Ohio 44313(US)

(72) Inventor: Lavanish, Jerome Michael
191 Harmony Hills Road
Akron Ohio 44321(US)

(74) Representative: Sternagel, Hans-Günther, Dr. et al,
Patentanwälte Dr. Michael Hann Dr. H.-G. Sternagel
Sander Aue 30
D-5060 Bergisch Gladbach 2(DE)

(54) **Herbicidal imidazolidinone derivatives.**

(57) The invention relates to certain 3-isoxazolyl- or 3-thiadiazol-2-imidazolidinone derivatives, namely 3-(5- or 3-substituted-3- or 5-isozazolyl)- or 3-(5-substituted-thiadiazol)-1-substituted-4- or 5-amino substituted -2-imidazolidinones and the use thereof for preemergence or postemergence control of noxious plants, i.e., weeds.

# HERBICIDAL IMIDAZOLIDINONE DERIVATIVES

## Field Of The Invention

This invention relates to certain 3-isoxazolyl- or 3-thiadiazol-2-imidazolidinone derivatives, namely 3-(5- or 3-substituted-3- or -5-isoxazolyl)-or 3-(5-substituted thiadiazol)-1-substituted-4- or 5-amino substituted -2-imidazolidinones and the use thereof for preemergence or postemergence control of noxious plants, i.e., weeds.

## Description of the Invention

This invention provides herbicidally active 3-(5- or 3-substituted-3- or -5-isoxazolyl)- or 3-(5- substituted-thiadiazol)-1-substituted-4- or 5-amino substituted -2-imidazolidinones represented by the Formula I:

I.

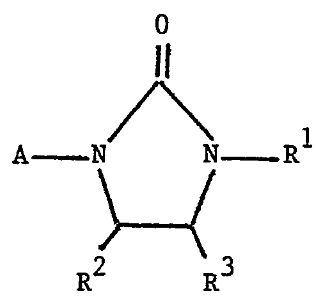

wherein A is:

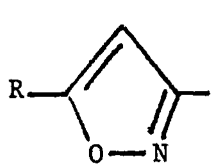 or 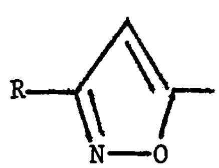 or 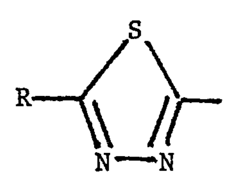 ;

wherein:

R is up to $C_6$ alkyl, haloalkyl or cycloalkyl, up to $C_5$ alkenyl or alkynyl, $-R^4-O-R^5$ or $-R^4-S-R^5$ wherein $R^4$ is up to $C_6$ alkylene and $R^5$ is up to $C_6$ alkyl optionally substituted phenyl or benzyl, alkylsulfonyl, amino or mono or dialkyl amino;

$R^1$ is up to $C_3$ alkyl or allyl;

$R^2$ and $R^3$ are selected from hydrogen, hydroxy or $-NR^6R^7$ with the proviso that one of $R^2$ or $R^3$ must $-NR^6R^7$, wherein $R^6$ is selected from hydrogen or up to $C_6$ alkyl or haloalkyl, benzyl or substituted benzyl; and $R^7$ is

$$\begin{array}{l} -CH-COOR^9 \\ \;\;| \\ \;\;R^8 \end{array}$$

wherein $R^8$ is selected from hydrogen, up to $C_6$ alkyl or haloalkyl, alkenyl, alkynyl, aryl or substituted aryl; and $R^9$ is selected from alkali metal, hydrogen, up to $C_6$ alkyl, haloalkyl or alkoxy-alkyl, phenyl or substituted phenyl.

The compounds of this invention can be synthesized using available starting materials, such as for example the compounds described in U. S. Patent Nos. 3,901,904; 3,920,674; 3,964,895; 4,012,223; 4,028,375; and 4,268,679; and using techniques known to the art. For example, certain of the compounds of this invention may be prepared by reacting a suitably substituted imidazolidinone compound of the formula:

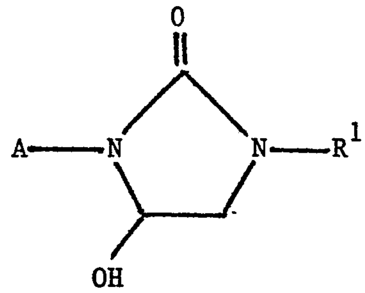

wherein A and $R^1$ are as previously defined, with a suitably substituted amine of the formula, $NHR^6R^7$, wherein $R^6$ and $R^7$ are as previously defined. The reaction is typically conducted in an inert organic solvent medium at up to reflux temperature and usually in the presence of a strong mineral or organic acid, e.g., p-toluene sulfonic acid.

The following Examples are illustrative of the preparation of certain compounds of this invention.

## Example I

Preparation of: 3-[5-(t-butyl)-1,3,4-thiadiazol-2-yl]-1-methyl-4-(N-carboethoxymethyl)amino-2-imidazolidinone

To a 50 milliliter flask provided with a Dean-Stark trap, a reflux condenser and a magnetic stirring bar were charged 0.5 gram (0.00195 mole) of 3-[5-(t-butyl)-1,3,4-thiadiazolyl]-1-methyl-4-hydroxy-2-imidazolidinone and 10 milliliters of dry toluene. To the resultant tan slurry was added 0.44 gram (0.0043 mole) of carboethoxymethyl amine followed by 0.1 gram of p-toluene sulfonic acid. The reaction mixture was heated to reflux and after a total of 8 hours reflux, since HPLC analysis indicated the presence of substantial unreacted starting material, an additional 0.4 gram of carboethoxymethyl amine was added and refluxing continued an additional 4 hours. The reaction mixture was then cooled, transferred to a separatory funnel containing 100 milliliters of saturated aqueous sodium bicarbonate solution and extracted with 3 x 120

milliliter portions of ethyl acetate. The combined organic layers were dried over anhydrous magnesium sulfate, filtered and the filtrate was concentrated in vacuo affording 0.76 gram of orange solid. Purification of this material by column chromatography on alumina afforded 0.45 gram of yellow oil confirmed by NMR analysis as the desired product.

## Example II

Preparation of: 3-[5-(t-butyl)-1,3,4-thiadiazol-2-yl]-1-methyl-4-[N-(1-carboethoxy)ethyl]amino-2-imidazolidinone

To a 50 milliliter flask provided with a Dean-Stark trap, a reflux condenser and a magnetic stirring bar were charged 0.5 gram (0.00195 mole) of 3-[5-(t-butyl)-1,3,4-thiadiazolyl]-1-methyl-4-hydroxy-2-imidazolidinone and 4 milliliters of dry toluene. To this stirred mixture was added 0.5 gram (0.0043 mole) of N-(1-carboethoxy) ethyl amine followed by 0.1 gram of p-toluene sulfonic acid and 4 milliliters of dry toluene. The mixture was heated to reflux and maintained at reflux for 8 hours at which time HPLC analysis indicated complete consumption of starting material. The reaction was then cooled, transferred to a separatory funnel containing saturated aqueous sodium bicarbonate solution and extracted with 3 x 110 milliliter portions of ethyl acetate. The combined organic layers were dried over anhydrous magnesium sulfate, filtered and the filtrate was concentrated in vacuo, affording 0.82 gram of golden oil confirmed by NMR analysis as the desired product.

## Example III

Preparation of: 3-[5-(t-butyl)-1,3,4-thiadiazol-2-yl]-1-methyl-4-[N-(1-t-butylcarboxy)ethyl]amino-2-imidazolidinone

To a 25 milliliter flask provided with a Dean-Stark trap, a reflux condenser and a magnetic stirring bar were charged 1.0 gram (0.0039 mole) of 3-[5-(t-butyl)-1,3,4-thiadiazolyl]-1-methyl-4-hydroxy-2-imidazolidinone and 8 milliliters of dry toluene. To this tan suspension was added 1.25 gram (0.0086 mole) of the L-isomer of N-(1-t-butylcarboxy)ethyl amine followed by 0.1 gram of p-toluene sulfonic acid. The reaction mixture was heated to reflux and maintained at reflux for 8 hours. The reaction mixture was the cooled, transferred to a separatory funnel, diluted with 100 milliliters of ethyl acetate and washed with 100 milliliters of saturated aqueous sodium bicarbonate solution. The organic layer was drawnoff and reserved and the aqueous phase was extracted with 3 x 100 milliliter portions of ethyl acetate. The organic extracts along with the reserved organic phase were combined, dried over anhydrous magnesium sulfate, filtered and the filtrate was concentrated in vacuo, affording 1.83 grams of a red oil confirmed by NMR analysis as desired product.

## Example IV

Preparation of: 3-[5-(t-butyl)-1,3,4-thiadiazol-2-yl]-1-methyl-4-[N-(1-t-butylcarboxy)methyl]amino-2-imidazolidinone

To a 50 milliliter flask provided with a Dean-Stark trap, a reflux condenser and a magnetic stirring bar were charged 1.0 gram (0.0039 mole) of 3-[5-(t-butyl)-1,3,4-thiadiazolyl]-1-methyl-4-hydroxy-2-imidazolidinone, 8 milliliters of dry toluene, 0.15 gram of p-toluene, 0.15 gram of p-toluene sulfonic acid and 0.67 gram (0.005 mole) of N-(1-t-butylcarboxy)methyl amine. The reaction mixture was heated to reflux and maintained at reflux for 4 hours. The reaction mixture was then cooled, transferred to a separatory funnel, diluted with 100 milliliters of ethyl acetate and washed with 100 milliliters of saturated aqueous sodium bicarbonate solution. The organic layer was drawn off, dried over anhydrous magnesium sulfate, filtered and the

filtrate was concentrated in vacuo affording 1.92 grams of golden oil, which after further reflux and isolation as described hereinabove afforded 1.84 grams of material confirmed by NMR analysis as the desired product.

Example V

Preparation of: 3-[5-(t-butyl)-3-isoxazolyl]-1-methyl-4-(N-carboethoxymethyl)amino-2-imidazolidinone.

To a 500 milliliter flask provided with a magnetic stirring bar, Dean-Stark trap and a reflux condenser were charged 19.0 grams (0.0794 mole) of 3[5-(t-butyl)-3-isoxazolyl]-1-methyl-4-hydroxy-2-imidazolidinone and 200 milliliters of dry toluene. This slurry was warmed on a steam bath until all of the solid dissolved. 18 grams (0.175 mole) of carboethyoxymethyl amine dissolved in 20 milliliters of dry toluene was added followed by 1.07 grams of p-toluene sulfonic acid. The reaction mixture was heated to reflux and maintained at reflux until HPLC analysis indicated complete conversion of starting materials. The reaction mixture was then cooled, transferred to a separatory funnel, diluted with 300 milliliters of ethyl acetate and washed with 300 milliliters of saturated aqueous sodium bicarbonate. The aqueous layer was drawn-off and extracted with 2x200 milliliter portions of ethyl acetate. The combined organic layers were dried over anhydrous magnesium sulfate, filtered and concentrated in vacuo affording 25.68 grams of reddish-orange oil confirmed by NMR analysis as the desired product.

Example VI

Preparation of: 3-[5-(t-butyl)-3-isoxazolyl]-1-methyl-4-(N carbomethoxymethyl)amino-2-imidazolidinone.

To a 100 milliliter flask provided with a magnetic stirring bar, a Dean-Stark trap and a reflux condenser were charged 7.17 grams (0.05 mole) of 3-[5-(t-butyl)-3-isoxazolyl]-1-methyl-4-hydroxy-2-imidazolidinone, and 40 milliliters of dry toluene. The mixture was heated via an oil bath until all of the solid dissolved. To the hot, yellow solution were added 3.8 grams (0.044 mole) of carbomethoxymethylamine and 0.52 grams of p-toluene sulfonic acid. The reaction was heated to reflux and maintained at reflux until TLC analysis indicated complete consumption of starting material. The reaction mixture was cooled, transferred to a separatory funnel, diluted with 100 milliliters of ethyl acetate and washed with a 120 milliliter portion of saturated aqueous sodium bicarbonate. The organic layer was washed with 100 milliliters of saturated brine and dried by filtering through a cone of anhydrous magnesium sulfate. The crude material was recrystallized from chloroform/hexane and solvent was removed in vacuo from the filtrate. The residue was digested with diethylether, concentrated to about 40 milliliters and cooled in an ice bath. The white precipitate was removed by filtration and the filtrate as stripped of solvent affording 7.4 grams of a golden oil confirmed by NMR analysis as the desired product.

Example VII

Preparation of: 3-[5-(t-butyl)-3-isoxazolyl]-1-methyl-4-[N-(1-carboethoxy)ethyl]amino-2-imidazolidinone.

To a 100 milliliter flask provided with a magnetic stirring bar, a Dean-Stark trap and reflux condenser were charged 4.78 grams (0.02 mole) of 3-[5-(t-butyl)-3-isoxazolyl]-1-methyl-4-hydroxy-2-imidazolidinone, and 20 milliliters of dry toluene. The mixture was heated via an oil bath to effect dissolution, after which were added 5.3 grams (0.0453

mole) of N-(1-carboethoxy)ethyl amine and 0.31 gram of p-toluene sulfonic acid. The reaction mixture was heated via the oil bath to 120°C. and maintained at that temperature for eight hours. After cooling to room temperature, HPLC analysis indicated that conversion of starting material was not quite complete, so heating at 120°C. was continued for another 16 hours. After cooling HPLC analysis indicated complete conversion of starting material. The reaction mixture was transferred to a separatory funnel, diluted with 100 milliliters of ethyl acetate and washed consecutively with 100 milliliter portions of saturated sodium bicarbonate and sodium chloride solutions. The organic layer was dried over anhydrous magnesium sulfate, filtered and concentrated in vacuo affording 6.45 grams of an orange oil confirmed by NMR analysis as the desired product.

Although the invention has been illustrated by the foregoing Examples with regard to the preparation of certain compounds within the scope of Formula I, it is to be understood that other compounds within the scope of Formula I may readily be prepared by those skilled in the art simply by varying the choice of starting materials and using the same or similar techniques.

Weed control in accordance with this invention is effected by applying to the soil prior to emergence of weeds therefrom or to the plant surfaces subsequent to emergence from the soil, a herbicidally effective amount of a compound of this invention. It is, of course, to be understood that the term "a compound of this invention" also includes mixtures of such compounds or a formulation containing a compound or mixture of compounds of this invention.

The term "herbicidally effective amount" is that amount of a compound of this invention required to so injure or damage weeds such that the weeds are incapable of recovering following application while not causing substantial injury to any valuable crop amongst which the weeds might be growing. The quantity of compound of this invention applied in order to exhibit a satisfactory herbicidal effect may vary over a wide range and depends on a variety of factors, such as, for example, hardiness of a particular weed species, extent of weed infestation, climatic conditions, soil conditions, method of application, and the like. Typically, as little as one or less pound or acre of a compound of this invention would be expected to provide satisfactory weed control, although in some instances application rates in excess of one pound per acre, e.g., up to 5 or more pounds per acre might be required. Of course, the efficacy of a particular compound against a particular weed species may readily be determined by routine laboratory or field testing in a manner well known to the art. It is expected that satisfactory weed control can be had at a rate of application in the range of 0.1 to 1.0 pound per acre.

Of course, a compound of this invention can be formulated according to routine methods with any of several known and commonly used herbicidal diluents, adjuvants and carriers. The formulations can contain liquid carriers and adjuvants such as organic solvents, as well as emulsifiers, stabilizers, dispersants, suspending agents, spreaders, penetrants, wetting agents and the like. Typical carriers utilized in dry formulations include clay, talc, diatomaceous earth, silica and the like. Preferred formulations are those in the form of wettable powders, flowables, dispersible granulates or aqueous emulsifiable concentrates

which can be diluted with water at the site of application. Also, dry formulations such as granules, dusts, and the like, may be used.

When, desired, a compound of this invention can be applied in combination with other herbicidal agents in an agents in an effort to achieve even broader vegetative control. Typical herbicides which can be conveniently combined with Formula I compound include atrazine, hexazinone, metribuzin, ametryn, cyanazine, cyprazine, prometon, prometryn, propazine, simazine, terbutryn, propham, alachlor, acifluorfen, bentazon, metalachlor and N,N-dialkyl thiocarbamates such as EPTC, butylate or venolate. These, as well as other herbicides described, for example, in the Herbicide Handbook of the Weed Science Society of America, may be used in combination with a compound or compounds of the invention. Typically such formulations will contain from about 5 to about 95 percent by weight of a compound of this invention.

The herbicidally formulations contemplated herein can be applied by an of several method known to the art. Generally, the formulation will be surfaced applied as an aqueous spray. Such application can be carried about by conventional ground equipment, or if desired, the sprays can be aerially applied. Soil incorporation of such surface applied herbicides is accomplished by natural leaching, and is of course facilitated by natural rainfall and melting snow. If desired, however, the herbicides can be incorporated into the soil by conventional tillage means.

Compounds of this invention are effective for preemergence or postemergence control of a wide variety of broadleaf and grassy weeds growning among corn or grain crops. Typical of the various species of

vegetative growth that may be controlled, combated, or eliminated are, for example, annuals such as pigweed, lambsquarters, foxtail, crabgrass, wild mustard, field pennycress, ryegrass, goose grass, chickweed, wild oats, velvetleaf, purslane, barnyardgrass, smartweed, knotweed, cocklebur, kochia, medic, ragweed, hemp nettle, spurrey, pondweed, carpetweed, morningglory, ducksalad, cheatgrass, fall panicum, jimsonweed, witchgrass, watergrass, wild turnip, and similar annual grasses and weeds. Biennials that may be controlled include wild barley, campion, burdock, bull thistle, roundleaved mallow, purple star thistle, and the like. Also controlled by the compounds of this invention are perennials such as quackgrass, Johnsongrass, Canada thistle, curley dock, field chickweed, dandelion, Russian knapweed aster, horsetail, ironweed, sesbania, cattail, wintercress, horsenettle, nutsedge, milkweed, sicklepod, and the like.

The compounds prepared as described in the Examples were individually tested for herbicidal efficacy against a variety of broadleaf and grassy weed species, under controlled laboratory conditions of light, humidity and temperature. Solvent solutions of said compounds were applied, both preemergence and postemergence, to test flats containing the various weed species, and herbicidal efficacy was evaluated vis a vis an untreated control, by periodic visual inspection after application of the compounds. Herbicidal efficacy was evaluated on a Numerical Injury Rating (NIR) scale of from 0 (no injury) to 10 (all plants dead). A NIR of 7-9 indicates severe injury; a NIR of 4-6 indicates moderate injury, i.e., plant growth is reduced to the extent that normal growth would be expected only under ideal conditions; and a NIR of 1-5 indicates slight injury.

The following table gives the average preemergence and postemergence NIR determined for each of the compounds prepared as described in Examples I through VII on the broadleaf (BL) and grassy (GR) weed species to which the compounds were applied. Each compound was applied at the indicated pound per acre rate and the NIR was determined three weeks subsequent to application.

| | I | II | III | IV | V | VI | VII |
|---|---|---|---|---|---|---|---|
| Pre-BL | 10 | 10 | 10 | 10 | 9.8 | 10 | 10 |
| Pre-GR | 10 | 9.5 | 9.6 | 10 | 9.2 | 8.3 | 10 |
| Post-BL | 9.3 | 9.6 | 9.1 | 3.9 | 9.4 | 9.2 | 9.8 |
| Post-GR | 5.0 | 3.8 | 3.8 | 0 | 6.5 | 6.5 | 7.0 |
| Rate | 1.0 | 1.0 | 1.0 | 1.0 | 0.5 | 0.5 | 0.5 |

The broadleaf weeds used in the screening tests were coffeeweed, jimsonweed, tall morningglory, teaweed, wild mustard, sicklepod, lambquarters and velvetleaf. The grassy weeds used in the screening tests were barnyardgrass, large crabgrass, Johnsongrass, wild oats and yellow foxtail.

Although the invention has been described in considerable detail by the foregoing, it is to be understood that many variations may be made therein by those skilled in the art without departing from the spirit and scope thereof as defined by the appended claims.

CLAIMS:

1. A compound of the formula:

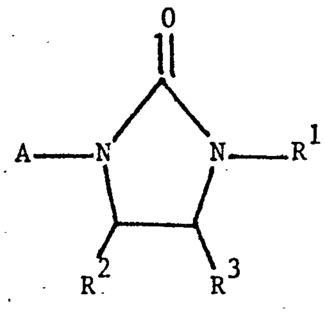

wherein A is:

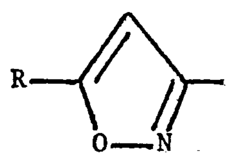 or 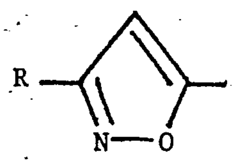 or 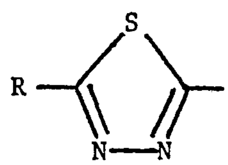 ;

wherein:

R is up to $C_6$ alkyl, haloalkyl or cycloaklyl, up to $C_5$ alkenyl or alkynyl; $-R^4-O-R^5$ or $-R^4-S-R^5$ wherein $R^4$ is up to $C_6$ alkylene and $R^5$ is up to $C_6$ alkyl optionally substituted phenyl or benzyl, alkyl sulfonyl, amino or mono or dialkylamino.

$R^1$ is up to $C_3$ alkyl or allyl;

$R^2$ and $R^3$ are selected from hydrogen, hydroxy or $-NR^6R^7$ with the proviso that one of $R^2$ or $R^3$ must $-NR^6R^7$, wherein $R^6$ is selected from hydrogen or up to $C_6$ alkyl or haloalkyl, benzyl or substituted benzyl; and $R^7$ is

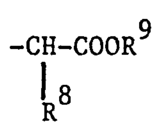

wherein $R^8$ is selected from hydrogen, up to $C_6$ alkyl or haloalkyl, alkenyl, alkynyl, aryl or substituted aryl; and $R^9$ is selected from alkali metal, hydrogen, up to $C_6$ alkyl, haloalkyl or alkoxy-alkyl, phenyl or substituted phenyl.

2. A compound of Claim 1 selected from 3-[5-(t-butyl)-1,3,4-thiadiazol-2-yl]-1-methyl-4-(N-carboethoxymethyl)amino-2-imidazolidinone; 3-[5-(t-butyl)-1,3,4-thiadiazol-2-yl]-1-methyl-4-[N-(1-t-butylcarboxy)ethyl]amino-2-imidazolidinone; 3-[5-(t-butyl)-1,3,4-thiadiazol-2-yl]-1-methyl-4-[N-(1-carboethoxy)ethyl]-amino-2-imidazolidinone; 3-[5-(t-butyl)-1,3,4-thiadiazol-2-yl]-1-methyl-4-[N-(1-t-butylcarboxy)methyl]amino-2-imidazolidinone; 3-[5-(t-butyl-3-isoxazolyl]-1-methyl-4-(N-carboethoxymethyl)amino-2-imidalidinone; 3-[5-(t-butyl)-3-isoxazolyl]-1-methyl-4-(N-carbomethoxymethyl)amino-2-imidazolidinone; or 3-[5-(t-butyl)-3-isoxazolyl]-1-methyl-4-[N-(1-carboethoxy)ethyl]amino-2-imidazolinone.

3. A herbicidal formulation containing an inert carrier and a herbicidally effective amount of a compound or mixture of compounds defined in Claim 1.

4. The method of controlling the growth of weeds wherein a herbicidally effective amount of herbicide is applied to a growth medium prior to emergence of weeds therefrom or to the weeds subsequent to emergence from the growth medium wherein the improvement resides in using as the herbicide a compound or mixture of compounds as defined in Claim 1.